# FASCICULE DE BREVET EUROPEEN

(11) **EP 0 538 303 B1**
(45) Date de publication et mention de la délivrance du brevet: **02.02.1994**
(21) Numéro de dépôt: 91912409.9
(22) Date de dépôt: 08.07.1991
(51) Int. Cl.: C07D 327/10

(54) **PROCEDE DE PREPARATION DE SULFATES CYCLIQUES**
VERFAHREN ZUR HERSTELLUNG VON CYCLISCHEN SULFATEN
METHOD FOR PREPARING CYCLIC SULPHATES

(30) Priorité: 09.07.1990 FR 9008686
(43) Date de publication de la demande: 28.04.1993
(73) Titulaire: RHONE-POULENC RORER S.A., 92165 Antony Cédex (FR)
(72) Inventeur: DERUELLE, Roger, F-94510 La Queue-en-Brie (FR); GUINARD, Michel, F-94320 Thiais (FR); PERRIER, Gérard, F-91000 Evry (FR)
(74) Mandataire: Pilard, Jacques
(86) Numéro de dépôt international: FR9100549
(87) Numéro de publication internationale: WO9200975

(56) Documents cités:
- EP-A- 0 343 053
- DE-A- 2 040 503
- US-A- 3 045 027
- US-A- 3 167 572

## Description

La présente invention concerne un procédé de préparation de sulfates cycliques de formule générale : 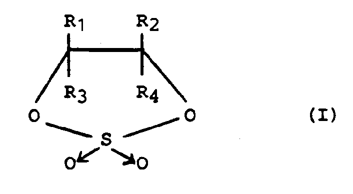
dans laquelle R₁, R₂, R₃ et R₄, identiques ou diférents, représentent un atome d'hydrogène ou un radical alcoyle contenant 1 à 4 atomes de carbone éventuellement substitué par un atome d'halogène.

Il est connu de préparer des sulfates cycliques par action de l'anhydride sulfurique sur un oxyde d'alcoylène en opérant dans le dioxanne (brevet américain US 3,045,027) ou dans un autre solvant organique tel que le dichloroéthane (brevets américains US 3,154,526 ou US 3,167,572) ou en phase gazeuse à une température inférieure à 140°C (brevet américain US 3,100,780). Cependant ces procédés ne permettent pas d'obtenir les sulfates cycliques avec des rendements satisfaisants.

Il a maintenant été trouvé, et c'est ce qui fait l'objet de la présente invention que les sulfates cycliques de formule générale (I) peuvent être obtenus avec des rendements généralement supérieurs à 80 % en ajoutant simultanément l'anhydride sulfurique et l'oxyde d'alcoylène dans le dioxanne éventuellement en présence d'un hydrocarbure aliphatique halogéné tel que le dichloro-1,2 éthane.

Pour la mise en oeuvre du procédé selon l'invention, il est particulièrement important d'opérer dans des conditions bien définies.

Plus précisément, il est nécessaire que le rapport molaire entre l'anhydride sulfurique et l'oxyde d'alcoylène soit maintenu à un valeur constante comprise entre 1,01 et 1,07 pendant toute la durée de l'addition. Il est avantageux de maintenir le rapport au voisinage de 1,04.

Généralement, on utilise un quantité de dioxanne telle que le rapport molaire entre le dioxanne et l'oxyde d'alcoylène mis en oeuvre soit compris entre 1 et 30. Lorque l'on utilise l'oxyde d'éthylène comme oxyde d'alcoylène, le rapport est de préférence voisin de 7.

La température de réaction est généralement comprise entre 30 et 60°C, de préférence entre 40 et 50°C.

Il est particulièrement avantageux d'utiliser du dioxanne anhydre qui peut être obtenu, par exemple, par distillation azéotropique avant l'introduction de l'anhydride sulfurique et de l'oxyde d'alcoylène.

L'anhydride sulfurique utilisé est de préférence de l'anhydride technique qui se présente sous forme liquide (pratiquement exempt de polymères linéaires et/ou réticulés)

L'oxyde d'alcoylène utilisé peut être introduit sous forme liquide ou gazeuse selon sa nature.

Le sulfate cyclique de formule générale (I) obtenu par la mise en oeuvre du procédé selon l'invention peut être soit utilisé tel quel après élimination du dioxanne par distillation rapide soit purifié par solubilisation, après "flash-distillation" du dioxanne, dans un solvant organique convenable tel qu'un hydrocarbure aliphatique halogéné comme le chlorure de méthylène suivie du lavage de la solution organique par de l'acide sulfurique éventuellement en solution aqueuse puis par l'eau jusqu'à neutralité : le sulfate cyclique est obtenu après évaporation du solvant.

Le procédé selon l'invention est particulièrement utile pour préparer le sulfate d'éthylène, le sulfate de propylène ou le sulfate de chlorométhyl-1 éthylène.

Les exemples suivants, donnés à titre non limitatif, montrent comment l'invention peut être mise en pratique.

### Exemple 1

Dans un réacteur en verre de 2,5 litres muni d'un agitateur, on introduit 2000 g (22,7 moles de dioxanne). On distille sous pression atmosphérique 500 g de dioxanne dans le but d'éliminer l'eau contenue dans le solvant. Après refroidissement à 45°C du dioxanne résiduel (1500 g ; 17,0 moles), on ajoute en parallèle 189 g d'anhydride sulfurique (2,36 moles) et 100 g d'oxyde d'éthylène (2,27 moles) en 100 minutes en respectant rigoureusement un rapport molaire anhydride sulfurique/oxyde d'éthylène égal à 1,04 et en maintenant la température à 45°C.

Après la fin de l'addition, le mélange réactionnel est agité pendant encore 30 minutes à 45°C.

Après refroidissement, le dosage du mélange réactionnel - par chromatographie à haute performance (CLHP) montre que le rendement en sulfate d'éthylène est de 90% par rapport à l'oxyde d'éthylène mis en oeuvre
- par chromatographie en phase gazeuse (CPG) montre que le taux de transformation de l'oxyde d'éthylène est de 100 %.

Après élimination du dioxanne par distillation sous pression réduite (20 mm/Hg ; 2,6 kPa), le sulfate d'éthylène brut dont la pureté est de 82% est extrait avec 1200 g de dichlorométhane. La solution chlorométhylénique est lavée à l'acide sulfurique concentré puis à l'eau et enfin est séchée sur sulfate de sodium. Après filtration et concentration à sec, on obtient 232 g de sulfate d'éthylène sous forme d'une poudre blanche fondant à 99°C et dont la pureté est de 97 %.

### Exemple 2

Dans un réacteur en verre de 2,5 litres muni d'un agitateur, on introduit 2.100 g de dioxanne (23,9 moles). On distille sous pression atmosphérique 300 g de dioxanne dans le but d'éliminer l'eau contenue dans le solvant. Après refroidissement du dioxanne résiduel (1800 g ; 20,5 moles) à 40°C, on introduit en parallèle 77,8 g d'anhydride sulfurique (0,97 moles) et 40 g d'oxyde d'éthylène (0,91 mole) en 60 minutes et en respectant rigoureusement un rapport molaire anhydride sulfurique/oxyde d'éthylène égal à 1,07 pendant toute la durée de l'addition et en maintenant la température à 40°C.

Après la fin de l'addition, le mélange réactionnel est agité pendant encore 30 minutes à 40°C.

Après refroidissement le dosage du mélange réactionnel : - par CLHP montre que le rendement en sulfate d'éthylène est de 95 % par rapport à l'oxyde d'éthylène mis en oeuvre,
- par CPG le taux de transformation de l'oxyde d'éthylène est de 100 %.

Après élimination du dioxanne par distillation sous pression réduite (20 mm/Hg ; 2,6 kPa), le sulfate d'éthylène brut dont la pureté est de 88 % est extrait avec 500 g de dichlorométhane. La solution chlorométhylénique est lavée à l'acide sulfurique concentré puis à l'eau jusqu'à neutralité et enfin séchée sur sulfate de sodium. Après filtration et élimination des solvants, on obtient 103,5 g de sulfate d'éthylène sous forme d'une poudre blanche fondant à 99° C et dont la pureté est de 97 %.

### Exemple 3

Dans un réacteur en verre de 2,5 litres muni d'un agitateur, on introduit 1800 g de dioxanne (20,5 moles). On distille sous pression atmosphérique 300 g de dioxanne dans le but d'éliminer l'eau contenue dans le solvant. Après refroidissement du dioxanne résiduel (1500 g ; 17,0 moles) à 45°C, on introduit en parallèle 189 g d'anhydride sulfurique (2,36 moles) et 132 g d'oxyde de propylène (2,26 moles) en 100 minutes et en respectant rigoureusement un rapport molaire anhydride sulfurique/oxyde de propylène égal à 1,04 pendant toute la durée de l'addition et en maintenant la température à 45°C.

Après la fin de l'addition, le mélange réactionnel est agité pendant encore 30 minutes à 45°C.

Après refroidissement, le dosage du mélange réactionnel par CLHP montre que le rendement en sulfate de propylène est de 76 % par rapport à l'oxyde de propylène mis en oeuvre.

Après traitement habituel, on obtient le sulfate de propylène dont le point d'ébullition est de 80°C sous une pression de 1 mm/Hg (0,13 kPa).

### Exemple 4

Dans un réacteur en verre de 2,5 litres muni d'un agitateur, on introduit 1800 g (20,5 moles) de dioxanne. On distille sous pression atmosphérique 300 g de dioxanne dans le but d'éliminer l'eau contenue dans le solvant. Après refroidissement du dioxanne résiduel (1500 g ; 17,0 moles) à 45°C, on introduit en parallèle 189 g d'anhydride sulfurique (2,36 moles) et 210 g d'épichlorhydrine (2,27 moles) en 100 minutes et en respectant rigoureusement un rapport molaire anhydride sulfurique/épichlorhydrine égal à 1,04 pendant toute la durée de l'addition et en maintenant la température à 45°C.

Après la fin de l'addition, le mélange réactionnel est agité pendant encore 30 minutes à 45°C.

Après refroidissement, le dosage du mélange réactionnel par CLHP montre que le rendement en sulfate de chlorométhyl-1 éthylène est de 79 % par rapport à l'épichlorhydrine mise en oeuvre.

Après traitement habituel, on obtient le sulfate de chlorométhyl-1 éthylène ayant les caractéristiques suivantes :
- spectre infra-rouge (en solution dans le dichlorométhane) : bandes d'absorption caractéristiques à 1398, 1214, 891, 651 et 535 cm⁻¹.
- spectre de masse (e.i.) : M/Z (%) = 172(8), 123(100), 137(5).

### Exemple 5

Dans un réacteur en verre de 2,5 litres muni d'un agitateur, on introduit 208 g (2,36 moles) de dioxanne et 1263 g de dichloro-1,2 éthane (1000 cm3). Le mélange réactionnel est chauffé à 45°C. On introduit en parallèle 189 g d'anhydride sulfurique (2,36 moles) et 132 g d'oxyde de propylène (2,26 moles) en 70 minutes et en respectant rigoureusement un rapport molaire anhydride sulfurique/oxyde de propylène égal à 1,04 pendant toute la durée de l'addition et en maintenant la température à 45°C.

Après la fin de l'addition, le mélange réactionnel est agité pendant encore 30 minutes à 45°C.

Après refroidissement, le dosage du mélange réactionnel par CLHP montre que le rendement en sulfate de propylène est de 84 % par rapport à l'oxyde de propylène mis en oeuvre.

### Exemple 6

Dans un réacteur en verre de 2,5 litres muni d'un agitateur, on introduit 208 g (2,36 moles) de dioxanne et 1263 g de dichlro-1,2 éthane (1000 cm3). Le mélange réactionnel est chauffé à 45°C. On introduit en parallèle 189 g d'anhydride sulfurique (2,36 moles) et 210 g d'épichlorhydrine (2,27 moles) en 69 minutes et en respectant rigoureusement un rapport molaire anhydride sulfurique/épichlorhydrine égal à 1,04 pendant toute la durée de l'addition et en maintenant la température à 45°C.

Après la fin de l'addition, le mélange réactionnel est agité pendant encore 30 minutes à 45°C.

Après refroidissement, le dosage du mélange réactionnel par CLHP montre que le rendement en sulfate de chlorométhyl-1 éthylène est de 87 % par rapport à l'épichlorhydrine mise en oeuvre.

## Revendications

1. Procédé de préparation d'un sulfate cyclique de formule générale 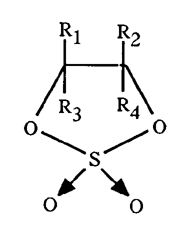 dans laquelle R₁, R₂, R₃ et R₄, identiques ou différents, représentent un atome d'hydrogène ou un radical alcoyle contenant 1 à 4 atomes de carbone éventuellement substitué par un atome d'halogène par action de l'anhydride sulfurique sur un oxyde d'alcoylène caractérisé en ce que l'on ajoute simultanément l'anhydride sulfurique et l'oxyde d'alcoylène dans le dioxanne éventuellement en présence d'un hydrocarbure aliphatique halogéné, étant entendu que le rapport molaire entre l'anhydride sulfurique et l'oxyde d'alcoylène est constant et compris entre 1,01 et 1,07 pendant toute la durée de l'addition.

2. Procédé selon la revendication 1 caractérisé en ce que le rapport molaire entre le dioxanne et l'oxyde d'alcoylène est compris entre 1 et 30.

3. Procédé selon l'une des revendications 1 ou 2 caractérisé en ce que l'on opère à une température comprise entre 30 et 60°C.

4. Procédé selon l'une des revendications 1 à 3 pour la préparation du sulfate d'éthylène, du sulfate de propylène et du sulfate de chlorométhyl-l éthylène.

## Claims

1. Process for the preparation of a cyclic sulphate of general formula 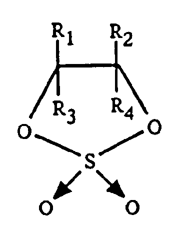 in which R₁, R₂, R₃ and R₄, which are identical or different, denote a hydrogen atom or an alkyl radical containing 1 to 4 carbon atoms, optionally substituted by a halogen atom, by reaction of sulphuric anhydride with an alkylene oxide, characterized in that sulphuric anhydride and the alkylene oxide are added simultaneously to dioxane, optionally in the presence of a halogenated aliphatic hydrocarbon, it being understood that, throughout the addition period, the molar ratio of sulphuric anhydride to the alkylene oxide is constant and is between 1.01 and 1.07.

2. Process according to Claim 1, characterized in that the molar ratio of dioxane to the alkylene oxide is between 1 and 30.

3. Process according to either of Claims 1 and 2, characterized in that the operation is carried out at a temperature of between 30 and 60°C.

4. Process according to one of Claims 1 to 3, for the preparation of ethylene sulphate, propylene sulphate and 1-chloromethylethylene sulphate.

## Patentansprüche

1. Verfahren zur Herstellung eines cyclischen Sulfats der allgemeinen Formel 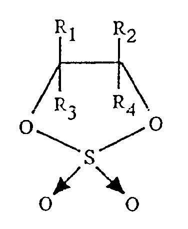 worin R₁, R₂, R₃ und R₄, identisch oder verschieden, ein Wasserstoffatom oder einen gegebenenfalls durch ein Halogenatom substituierten Alkylrest mit 1 bis 4 Kohlenstoffatomen bedeuten, durch Umsetzung von Schwefelsäureanhydrid mit einem Alkylenoxid, dadurch gekennzeichnet, daß man gleichzeitig das Schwefelsäureanhydrid und das Alkylenoxid in Dioxan, gegebenenfalls in Gegenwart eines halogenierten aliphatischen Kohlenwasserstoffs, zugibt, wobei das Molverhältnis zwischen dem Schwefelsäureanhydrid und dem Alkylenoxid konstant ist und zwischen 1,01 und 1,07 während der gesamten Zugabedauer beträgt.

2. Verfahren gemäß Anspruch 1, dadurch gekennzeichnet, daß das Molverhältnis zwischen dem Dioxan und dem Alkylenoxid zwischen 1 und 30 liegt.

3. Verfahren gemäß einem der Ansprüche 1 oder 2, dadurch gekennzeichnet, daß man bei einer Temperatur zwischen 30 und 60°C arbeitet.

4. Verfahren gemäß einem der Ansprüche 1 bis 3 zur Herstellung von Ethylensulfat, Propylensulfat und 1-Chlormethylethylensulfat.
